(19) Europäisches Patentamt

European Patent Office

Office européen des brevets

(11) **EP 1 036 542 B1**

(12) **EUROPEAN PATENT SPECIFICATION**

(45) Date of publication and mention
of the grant of the patent:
**26.11.2003 Bulletin 2003/48**

(51) Int Cl.[7]: **A61B 6/00**

(21) Application number: **99105250.7**

(22) Date of filing: **15.03.1999**

(54) **Apparatus for measuring hepatic blood flow amount**

Gerät zur Messung des Blutflusses der Leber

Appareil de mesure de débit sanguin du foie

(84) Designated Contracting States:
**DE FR GB SE**

(43) Date of publication of application:
**20.09.2000 Bulletin 2000/38**

(73) Proprietor: **ANZAI MEDICAL KABUSHIKI KAISHA
Tokyo 141-0033 (JP)**

(72) Inventor: **Sase, Shigeru c/o ANZAI MEDICAL K. K.
Tokyo 141-0033 (JP)**

(74) Representative: **Klunker . Schmitt-Nilson . Hirsch
Winzererstrasse 106
80797 München (DE)**

(56) References cited:
**EP-A- 0 370 636          EP-A- 0 551 898
DE-A- 3 522 113**

• **GUR ET AL. : "Blood flow mapping in the human
liver by the xenon/ct method" JOURNAL OF
COMPUTER ASSISTED TOMOGRAPHY, vol. 9,
no. 3, 1985, pages 447-450, XP002112960**
• **TANIGUCHI ET AL.: "Analysis of models for
quantification of arterial and portal blood flow in
the human liver using PET" JOURNAL OF
COMPUTER ASSISTED TOMOGRAPHY, vol. 20,
no. 1, 1996, pages 135-144, XP002112961**

**Description**

BACKGROUND OF THE INVENTION

Field of the Invention:

The present invention relates to a method and an apparatus for measuring the hepatic blood flow amount. In particular, the present invention relates to a method and an apparatus for measuring the hepatic blood flow amount, which make it possible to easily measure the blood flow amount concerning hepar (hereinafter simply referred to as "liver" as well) by utilizing, a xenon gas inhalator and an X-ray CT (computerized tomography) system.

Description of the Related Art:

A method has been hitherto known, in order to measure the blood flow in the head of a patient. In this method, for example, a mixed gas of xenon gas and oxygen gas, which is fed from a gas inhalator, is allowed to be inhaled by the patient for a certain period of time by using a respiratory mask followed by allowing the patient to inhale the normal air, while periodically obtaining tomographic images of the head of the patient as a specimen by using an X-ray CT system, to analyze the tomographic images so that the blood flow in the head of the patient is measured.

That is, according to the measuring method, the mixed gas passes through the lung of the patient, and it is absorbed into the pulmonary artery. The mixed gas passes through the heart, and it flows as an arterial blood flow into the tissue of the head. The mixed gas passes through the head tissue, and it is returned through the venous blood flow to the heart. The mixed gas passes through the heart, and it is returned to the pulmonary vein. During this process, the time-dependent change in xenon concentration in the head tissue is observed by using the X-ray CT system. The observed time-dependent change is compared with an authentic time-dependent change in xenon concentration in the head in which the tissue is normal. Thus, it is possible to diagnose the head of the patient.

The method for measuring the blood flow based on the use of the xenon gas as described above is not limited to the diagnosis of the head, and it can be also applied to normal organs (internal organs) such as the stomach, the intestine, and the pancreas in which the arterial blood flow inflows thereinto and the venous blood flow outflows therefrom as in the head.

However, when it is intended that the method for measuring the blood flow described above is applied to the liver, it is noted that not only the arterial blood flow inflows into the liver. As disclosed in "EIZOJOHO MEDICAL" {issued in April 1994, Volume 26, Number 8, "Method for simultaneously measuring blood flows in hepatic artery and portal vein based on $H_2^{15}O$ intravenous injection method by using positron CT (about local blood flow amount difference in liver region)" (page 449 to page 453, Hiroki TANIGUCHI et al., the First Department of Surgery of Kyoto Prefectural University of Medicine) published by Industrial Development Organization (SANGYO KAIHATSUKIKO INC.) Co., Ltd.), it is known that the portal blood flow as the venous blood flow, which outflows from the stomach, the intestine, the pancreas, and the spleen as the portal internal organs, also inflows into the liver. Therefore, it is impossible to apply the foregoing conventional technique to the measurement of the blood flow amount in the liver.

A method for measuring the blood flow amount in the liver is disclosed in "EIZOJOHO MEDICAL" described above. That is, a principle expression is introduced for the amount of blood which inflows and outflows with respect to the liver. The principle expression is solved on the basis of the result of measurement based on the $H_2^{15}O$ intravenous injection method by using the positron CT. Further, the liver is divided into four regions for each of which the total hepatic blood flow amount, the portal blood flow amount (total amount), and the hepatic arterial blood flow amount (total amount) can be measured concerning an injured liver and a non-injured liver.

However, in the case of the intravenous injection method based on the use of the positron CT, even when a blood flow image is obtained, then it is difficult to make comparison with an anatomical structure, and hence it is difficult to make application to the image diagnosis.

"Taniguchi et al. (1996): 'Analysis of models for quantification of arterial and portal blood flow in human liver using PET' (see European Search Report)" discloses a method to quantify arterial and portal hepatic arterial blood flows. Simultaneous measurement of hepatic arterial and portal blood flow is possible using the radioactive Xenon method and catheter technique. A method is referred to that quantifies hepatic blood flow by PET. Regarding the regional arterial hepatic blood flow, the regional portal hepatic blood flow, the tracer concentration in the arterial blood, and the coefficients for the portal organ and the liver, there is calculated the radioactivity in the liver that is washed out by hepatic venous flow.

"Gur et al. (1985):'Blood flow mapping in the human liver by the xenon/ct method' (see European Search Report)" discloses an apparatus for measuring a hepatic blood flow amount, comprising a gas inhalator for supplying a mixture of xenon gas and oxygen to a lung of a specimen and an X-ray CT system for detecting a change in xenon concentration in a tissue of a liver. This apparatus is described as a prototype xenon/CT inhalation system. The method describes the use of two-second scans of the liver and breath-halting in all studies. Furthermore, all studies were performed during inhalation of a 33% xenon/oxygen mixture, with a variable number of breaths separating each imaging period. Because relatively high blood flow values in the organs of interest (liver and possibly the spleen) were anticipated, scanning at the first level was begun approximately 25 s after the start of xenon inhalation and continued for approximately 3.5 min.

## SUMMARY OF THE INVENTION

The present invention has been made considering the knowledge and the problem as described above, an object of which is to provide an apparatus for measuring the hepatic blood flow amount, which make it possible to measure the hepatic arterial blood flow amount and the portal blood flow amount in a form suitable for diagnosis.

Another object of the present invention is to provide an apparatus for measuring the hepatic blood flow amount, which make it possible to detect the portal blood flow amount and the arterial blood flow amount for each of the tissues of the liver.

The above and other objects, features, and advantages of the present invention will become more apparent from the following description when taken in conjunction with the accompanying drawings in which a preferred embodiment of the present invention is shown by way of illustrative example.

## BRIEF DESCRIPTION OF THE DRAWINGS

FIG. 1 shows a schematic perspective view illustrating an overall arrangement of an embodiment of the present invention;

FIG. 2 shows a block diagram illustrating a system of the embodiment of the present invention;

FIG. 3 shows a flow chart to be used to explain the operation of the embodiment of the present invention;

FIG. 4 shows a schematic side view illustrating a state in which a liver of a patient as a specimen supplied with xenon gas is photographed by using an X-ray CT system;

FIG. 5 shows a schematic diagram of the liver for the purpose of computer analysis;

FIG. 6 shows a monochromatic illustration depicting a tomographic image of a portion including the liver;

FIG. 7 shows a characteristic curve illustrating the change in xenon concentration in the main artery;

FIG. 8 shows a characteristic curve illustrating the change in xenon concentration in a certain region of interest;

FIG. 9 shows a characteristic curve illustrating the change in xenon concentration in another region of interest;

FIG. 10 shows a characteristic curve obtained by making approximation for the characteristic curve shown in FIG. 8;

FIG. 11 shows a characteristic curve obtained by making approximation for the characteristic curve shown in FIG. 9;

FIG. 12 shows a color illustration depicting an artery map display of the liver;

FIG. 13 shows a color illustration depicting a portal map display of the liver;

FIG. 14 shows a color illustration depicting a hepatic blood flow map of the liver; and

FIG. 15 shows a flow chart to be used to explain the operation of another embodiment of the present invention.

## DESCRIPTION OF THE PREFERRED EMBODIMENTS

An embodiment of the present invention will be explained below with reference to the drawings.

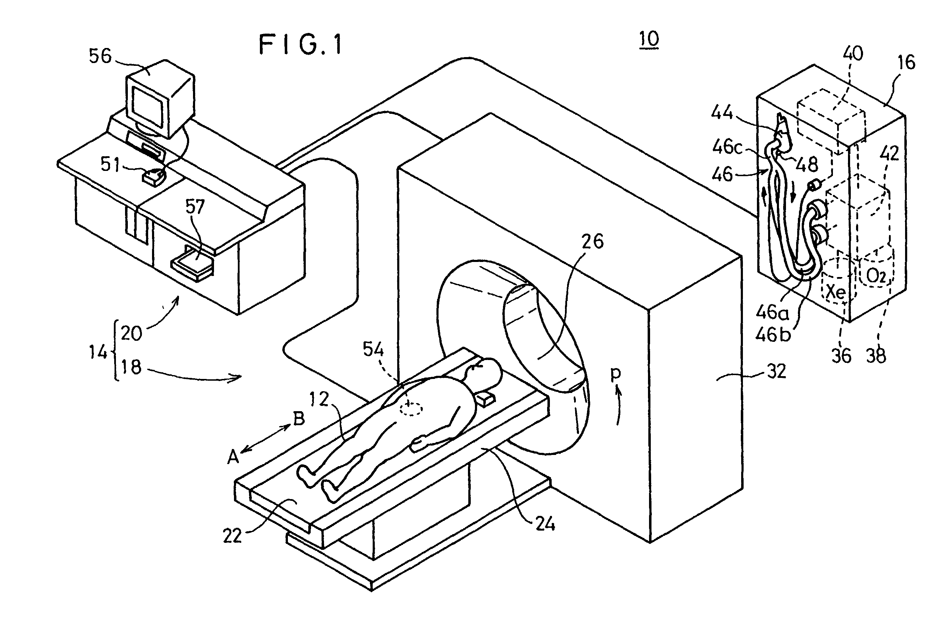

FIG. 1 shows a an overall arrangement of an apparatus 10 for measuring the hepatic blood flow amount according to the embodiment.

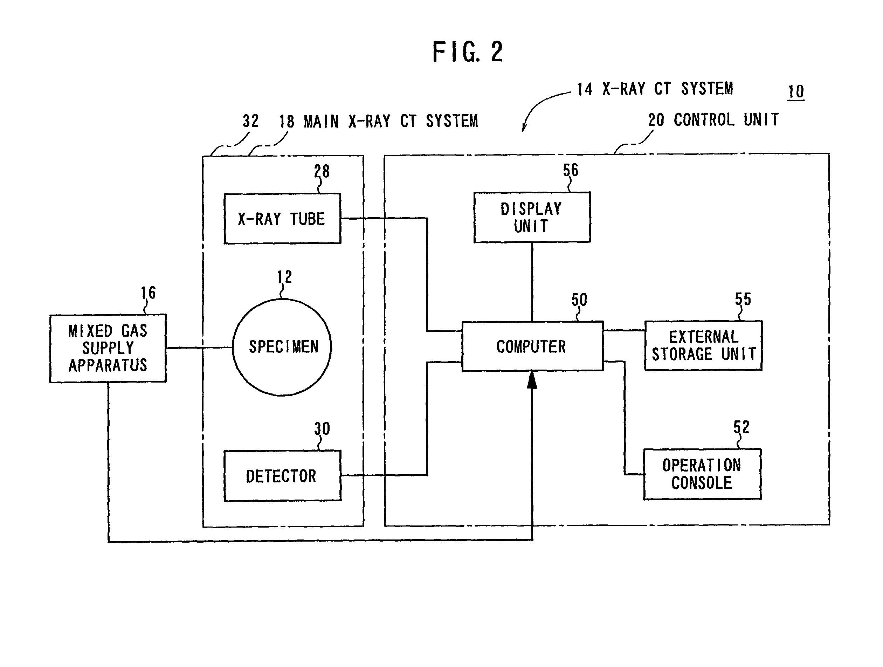

FIG. 2 shows a block diagram of the apparatus 10 for measuring the hepatic blood flow amount shown in FIG. 1.

With reference to FIGS. 1 and 2, the apparatus 10 for measuring the hepatic blood flow amount basically comprises an X-ray CT system 14 for obtaining a tomographic image of a specimen 12 such as human, and a mixed gas supply apparatus 16 for supplying a mixed gas of xenon (Xe) and oxygen ($O_2$) to the specimen 12. The X-ray CT system 14 comprises a main X-ray CT system 18, and a control unit 20 for controlling the main X-ray CT system 18 and for controlling the mixed gas supply apparatus 16. The control unit 20 also functions as a data-processing unit for processing image data or the like obtained by using the main X-ray CT system 18. The control unit 20 may be arranged such that it is physically divided into a control unit for controlling the main X-ray CT system 18 and a control unit for controlling the mixed gas supply apparatus 16.

The main X-ray CT system 18 is provided with a specimen-placing stand 24 which is arranged, on its top surface, with a movable table 22 for placing the specimen 12 thereon to be moved in directions indicated by the arrows A and B, and a gantry 32 which is formed with a cylindrical opening 26. The gantry 32 is arranged with an X-ray tube 28 (see FIG. 2) which revolves around the cylindrical opening 26 in a direction indicated by the arrow p (see FIG. 1), and a detector 30 which is composed of a plurality of detector elements disposed on the circumference around the opening 26.

The mixed gas supply apparatus 16 includes a xenon gas cylinder 36, an oxygen gas cylinder 38, a main inhalator body 42 for mixing the gases under the control of an internal computer 40, and a conduit 46 having one end connected to the main inhalator body 42 and the other end connected to a respiratory mask 44. In this embodiment, the conduit 46 comprises an inspiration tube 46a, an expiration tube 46b, and a respiratory mask conduit 46c. A xenon gas concentration-measuring sensor 48 is attached to the respiratory mask 44. A detection signal obtained by the concentration-measuring sensor 48 is supplied to the computer 40 so that the computer 40 calculates the xenon gas concentration in the expiration gas. The computer 40, which controls the overall operation of the mixed gas supply apparatus 16, is electrically connected to the control unit 20 so that they communicate with each other.

As shown in FIG. 2, the control unit 20 includes a computer 50 which functions as a control unit and a processing unit. The computer 50 is used to control the operation of the main X-ray CT system 18 and the mixed gas supply apparatus 16. The computer 50 also processes the picture element data for constructing the tomographic image of the hepar 54 (hereinafter referred to as "liver" as well) of the specimen 12 detected by the detector 30 disposed in the gantry 32 to prepare, for example, the tomographic image. The computer 50 is further connected with an operation console 52 including a mouse 51 and a keyboard, an external storage unit 55 such as a magneto-optical disk unit, and a display unit 56 such as a color CRT (cathode ray tube). In the illustrative apparatus 10 for measuring the hepatic blood flow amount shown in FIGS. 1 and 2, the operation console 52 is practically used such that a mouse pointer, which is displayed on the screen of the display unit 56 and which is operated by using the mouse 51, is used to click the concerning display on the screen so that the process indicated by the display is executed. As described later on, for example, the tomographic image (so-called CT image) of the liver 54 represented by the picture element data, which is obtained by the main X-ray CT system 18 by the aid of the computer 50, is displayed in color or monochromatic illustration on the display unit 56. The image of the liver 54 of the specimen 12, which is subjected to classification into those concerning the tissue through which the hepatic arterial blood flow flows and those concerning the tissue through which the portal blood flow flows, is also displayed on the display unit 56. The image, which is displayed on the screen of the display unit 56, can be printed out by using a printer contained in the control unit 20 so that the image is outputted as a color or monochromatic hard copy 57 (see FIG. 1).

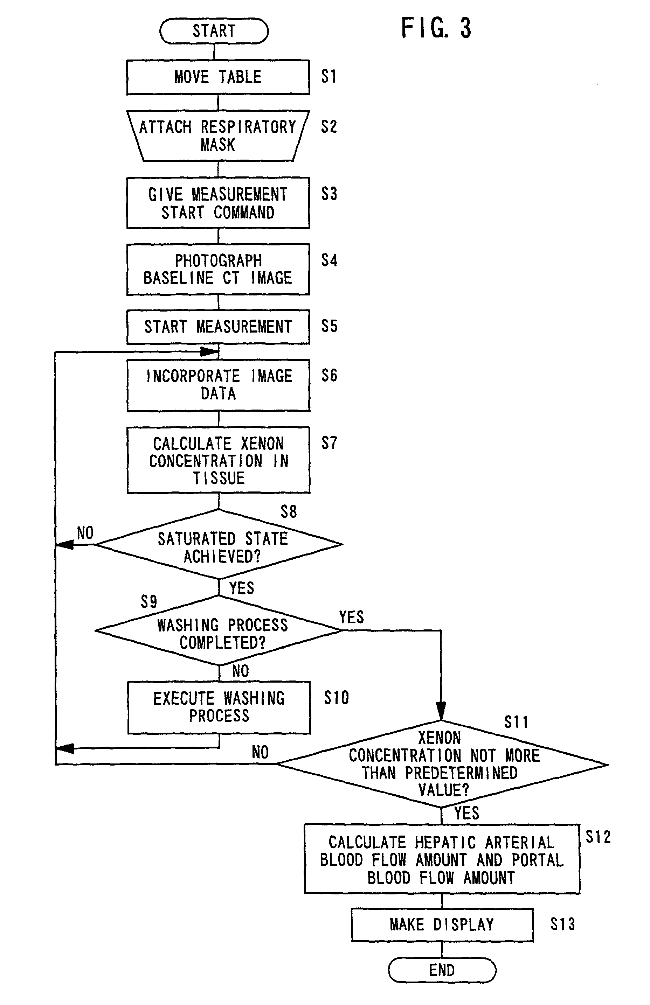

Next, the operation of the embodiment described above will be explained on the basis of a flow chart shown in FIG. 3. The control entity for the flow chart is the computer 50.

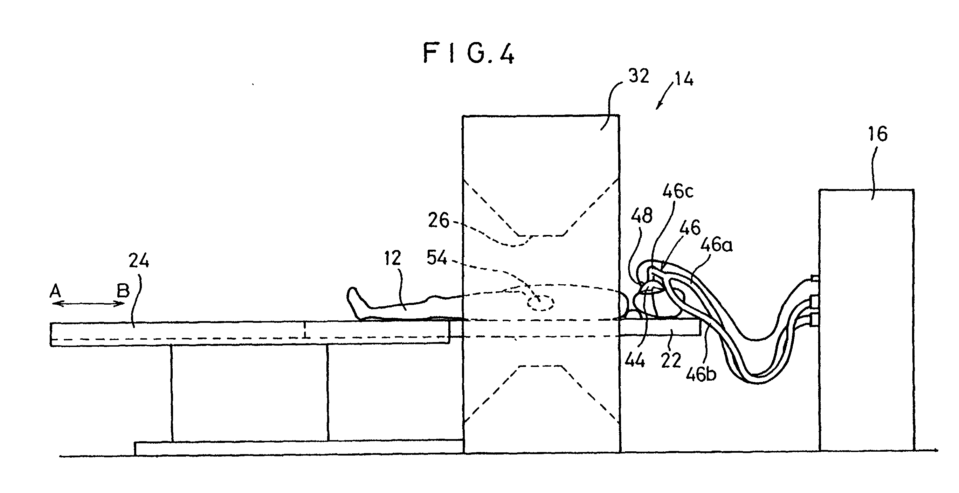

At first, an operator such as a medical doctor operates the operation console 52 so that the movable table 22 is moved in the direction of the arrow B in a state in which the specimen 12 is placed on the specimen-placing stand 24 as shown in FIG. 4. The movable table 22 is stopped at a position at which the liver 54 of the specimen 12 can be photographed to obtain the tomographic image (step S1).

Subsequently, as shown in FIG. 4, the respiratory mask 44 is attached so that the mouth and the nose of the specimen 12 are covered therewith (step S2).

The operation console 52 is operated in the measurable state as shown in FIG. 4. Thus, a measurement start command is sent from the computer 50 of the control unit 20 to the computer 40 of the mixed gas supply apparatus 16, and it is sent to the main X-ray CT system 18 (step S3).

During this process, the tomographic image of the liver 54, i.e., the so-called baseline CT image is photographed by using the main X-ray CT system 18, and it is incorporated into the external storage unit 55 (step S4).

Subsequently, the xenon gas and the oxygen gas, which are fed from the xenon gas cylinder 36 and the oxygen gas cylinder 38 respectively, are mixed in a ratio of 30 % of the xenon gas and 70 % of the oxygen by using the main inhalator body 42 under the control of the computer 40 of the mixed gas supply apparatus 16. The mixed gas is allowed to pass through the inspiration tube 46a, the respiratory mask conduit 46c, and the respiratory mask 44, and it is supplied to the lung of the specimen 12. The expiration gas, which is discharged from the lung of the specimen 12, passes through the respiratory mask 44, the respiratory mask conduit 46c, and the expiration tube 46b, and it is returned to the main inhalator body 42. The measurement is started while controlling the mixed gas supply apparatus 16 by the aid of the computer 40 so that the xenon gas concentration in the mixed gas has a predetermined value (30 % in this embodiment) from the point of time of the start of the supply of the mixed gas to the specimen 12. Thus, the inhalation process, i.e., so-called Wash-in is started (step S5). For example, an apparatus disclosed by the present applicant in Japanese Patent Publication No. 3-33326 can be used as the mixed gas supply apparatus 16. The xenon gas concentration in the expiration gas is measured at every 40 msec from the point of time of the start of the measurement.

The X-ray is radiated onto the specimen 12 from the X-ray tube 28 in the gantry 32 at about every 60 seconds while performing the process to make change to the saturation judgement, the washing process, or the so-called Wash-out process as described later on, starting from the point of time of the start of the inspiration of the mixed gas with respect to the specimen 12. The X-ray, which has passed through the specimen 12, is detected by the detector 30. Thus, the tomographic image of the liver 54 is photographed at about every 60 seconds, and it is incorporated as picture element data into the computer 50 (step S6).

Subsequently, the xenon concentration in the liver tissue is calculated for each of the picture elements on the basis of the picture element data (step S7). In this embodiment, the size of the picture element is about 0.5 mm square. However, the size may be changed into an appropriate size. In order to easily understand the present invention, it is conveniently assumed that the phrase "each picture element" referred to in the following description has the same meaning as that of the phrase "each tissue or each individual tissue" for constructing the liver 54.

Accordingly, it is possible to obtain the xenon concentration Ch(t) of each tissue of the liver 54 of the specimen 12 {t represents the measurement time (second), and it has values of about every 60 seconds, for example, t = 0, 62, 123, 183, 243, 303, 363, 424, 484, 544,...}.

If the rate of increase in the xenon concentration Ch(t) in the liver tissue is smaller than a predetermined value having been previously prescribed, then it is judged that the saturated state is achieved (step S8). After it is judged whether or not the washing process is completed (step S9), the supply of the mixed gas is stopped to perform the so-called Wash-out in which the normal air is fed in place of the mixed gas (step S10).

Further, the process of the step S6 is performed at about every 1 minute, and the process of the step S7 is performed at every 40 ms to complete the washing process. After that (after the judgement in the step S9 is affirmative), the process of the step S6 is performed at about every 1 minute, and the process of the step S7 is performed at every 40 ms in the same manner as described above until the xenon concentration Ch(t) in the liver tissue is not more than the predetermined value (step S11). If the xenon concentration Ch(t) is not more than the predetermined value (step S11: YES), the hepatic arterial blood flow amount and the portal blood flow amount are calculated as explained below (step S12) on the basis of the concentration data of the expiration gas determined by using the xenon gas concentration-measuring sensor 48 and the xenon concentration Ch(t) of the liver 54. Various displays are made on the display unit 56 on the basis of the results of the calculation and other factors as described later on (step S13).

Next, detailed explanation will be made below for the process of the step S12 and the followings for calculating the hepatic arterial blood flow amount and the portal blood flow amount for each of the tissues of the liver 54.

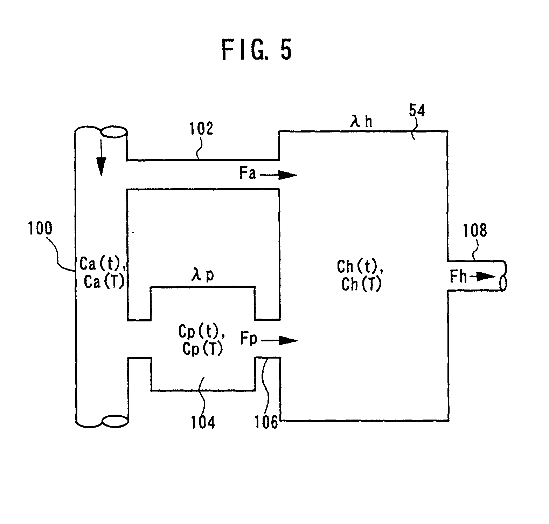

FIG. 5 shows a known schematic diagram in which the blood flow supplied to the liver 54 is simplified (for example, see "EIZOJOHO MEDICAL" described above). As understood from FIG. 5, the hepatic arterial blood flow amount Fa is supplied to each of the tissues of the liver 54 from the main artery 100 via the hepatic artery 102. The portal blood flow amount Fp is supplied to each of the tissues of the liver 54, which resides in the venous blood flow fed from the main artery 100 via the portal vein 106 after passing through the portal internal organs 104 such as the stomach, the intestine, the pancreas, and the spleen. In this case, the ratio of the hepatic arterial blood flow amount Fa to the portal blood flow amount Fp is Fa/Fp ≈ 1/3 in average. The hepatic arterial blood flow amount Fa and the portal blood flow amount Fp pass through the tissue of the liver 54, and they are fed as the hepatic venous blood flow amount Fh to the hepatic vein 108. The unit of each of the blood flow amounts Fa, Fp, Fh is the amount (cc) of blood flowing through the unit volume (100 cc) per 1 minute.

The relationship represented by the following expression (1) holds concerning the respective blood flow amounts Fa, Fp, Fh.

$$Fh = Fa + Fp \qquad (1)$$

As described above, the xenon concentration Ch(t) has been obtained for each of the tissues of the liver 54 of the specimen 12 by using the X-ray CT system 14.

The xenon concentration Ca(t) in the blood flow in the main artery 100 shown in FIG. 5, i.e., the xenon concentration in the arterial blood flow flowing into the hepatic arterial blood flow amount Fa and the portal internal organs 104 is deduced from the xenon concentration in the end-expiration gas, and hence it can be calculated from the xenon concentration having been measured by using the concentration-measuring sensor 48.

On this assumption, it has been revealed that the time-dependent change in xenon concentration Ch(t) of each tissue of the liver 54 resides in the Fick's principle as represented by the following expression (2), and thus it is represented by the value obtained such that the product of the hepatic venous blood flow amount Fh, the xenon concentration Ch(t) of each liver tissue, and the reciprocal (1/λh) of the partition coefficient is subtracted from the sum of the product of the hepatic arterial blood flow amount Fa and the xenon concentration Ca(t) in the hepatic arterial blood flow and the product of the portal blood flow amount Fp, the xenon concentration Cp(t) in the portal internal organs 104, and the reciprocal (1/λp) of the partition coefficient.

$$dCh(t)/dt = Fa \cdot Ca(t) + Fp \cdot Cp(t)/\lambda p - Fh \cdot Ch(t)/\lambda h \qquad (2)$$

The partition coefficients λp and λh represent the ratios between the xenon concentration in the tissue and the xenon concentration in the blood in the portal internal organs 104 and the liver 54 respectively.

Concerning the expression (2), the xenon concentration Cp(t) in the portal internal organs 104 cannot be measured, and hence it should be artificially eliminated from the expression (2).

In this case, it has been revealed that the following expression (3) is obtained according to the Fick's principle taking the portal internal organs 104 into consideration. That is, the time-dependent change of the xenon concentration Cp(t) of the portal internal organs 104 is represented by the value obtained such that the product of the portal blood flow amount Fp, the xenon concentration Cp(t) of the portal internal organs 104, and the reciprocal of the partition coefficient (1/λp) is subtracted from the product of the portal blood flow amount Fp and the xenon concentration Ca(t) in the hepatic arterial blood flow.

$$dCp(t)/dt = Fp \cdot Ca(t) - Fp \cdot Cp(t)/\lambda p \tag{3}$$

When the expression (3) is solved for the xenon concentration Cp(t) in the portal internal organs 104, it is understood that the xenon concentration Cp(t) is represented by the following expression (4) containing the definite integral. In the expression (4), the symbol x represents the time.

$$Cp(t) = Fp \cdot \int Ca(x) \cdot \exp\{(-Fp/\lambda p)(t-x)\}dx \tag{4}$$

In the expression (4), the domain of the definite integral is [0, t].

The expression (2) is substituted with the expression (4) to determine the xenon concentration Ch(T) of the liver tissue. Thus, as shown in the following expression (5), it is deduced that the xenon concentration Ch(T) is represented by the sum of the definite integrals.

$$Ch(T) = Fa\int Ca(t) \cdot \exp\{(-Fh/\lambda h)(T-t)\}dt$$

$$+ Fp(Fp/\lambda p)\int[\int Ca(x) \cdot \exp\{(-Fp/\lambda p)(t-x)\}dx] \cdot$$

$$\exp\{(-Fh/\lambda p)(T-t)\}dt \tag{5}$$

In the expression (5), the interval of the definite integral of the first term on the right side is [0, T]. In the double integral of the second term on the right side, the interval of the definite integral for the infinitesimal time dx is [0, t], and the interval of the definite integral for the infinitesimal time dt is [0, T].

In the expression (5), the approximate solutions for the hepatic arterial blood flow amount Fa and the portal blood flow amount Fp can be determined for each tissue (each picture element) of the liver 54 by means of the least square method while assuming that there is given λh = λp. The ratio of the flows can be calculated for each tissue. The portion, in which the value of the ratio satisfies Fa/Fp ≈ 1/3, is considered to be highly possibly a normal tissue. The portion, in which the value of the ratio is deviated to increase or decrease, is considered to be highly possibly an abnormal tissue. In general, it is acknowledged that there is no tissue in which only the arterial blood flow flows. Therefore, the tissue (site), in which only the arterial blood flow flows, can be estimated, for example, to be a tissue (site) in which any abnormal tissue such as malignant tumor exists.

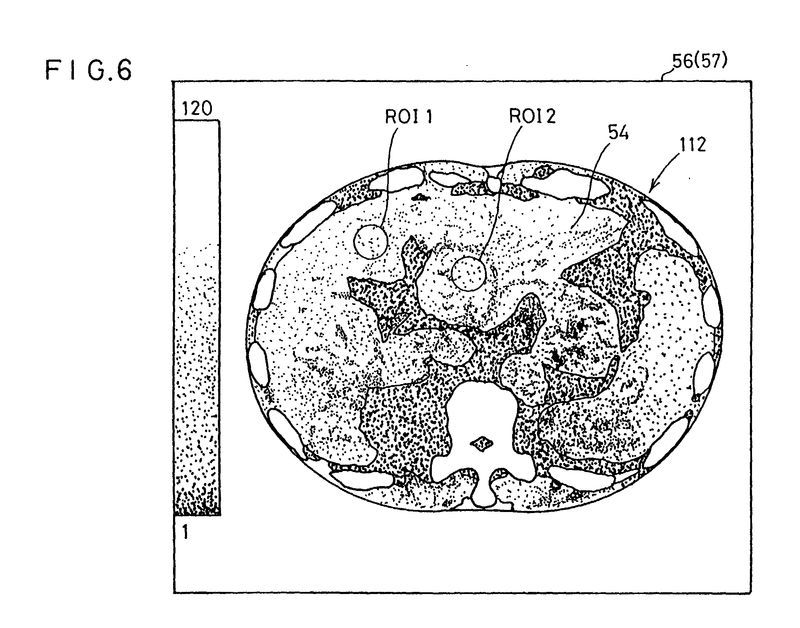

The analysis of the region of interest ROI in the liver 54 will be specifically explained below. FIG. 6 shows an output image 112 displayed in monochromatic illustration on the display unit 56 or on the hard copy 57 obtained by using the X-ray CT system 14, the output image 112 including the tomographic image of the liver 54 photographed in the step S4. In FIG. 6, the indication ranging from a value of 1 to a value of 120 depicted at the left end represents the CT value, i.e., the value of the Hounsfield unit [HU]. For example, two region of interest ROI1 and ROI2, which are now intended to be analyzed, are set in desired sites (positions) of the liver 54 in the output image 112 by using the operation console 52 (see the positions (regions) indicated by circles in FIG. 6). The region of interest ROI can be set in an arbitrary size and at an arbitrary position.

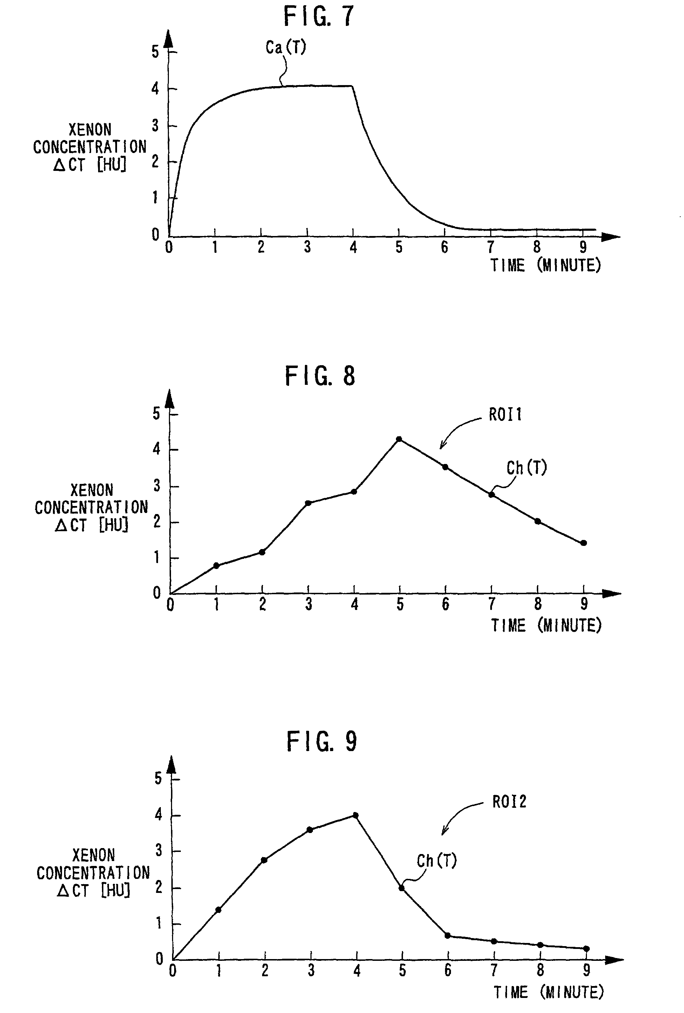

FIG. 7 shows the change (measured value) of the xenon concentration Ca(T) in the main artery 100 measured on the basis of the xenon concentration in the expiration gas. FIG. 8 shows an example of the change (measured value) of the xenon concentration Ch(T) of the liver tissue of the region of interest ROI1. FIG. 9 shows an example of the change (measured value) of the xenon concentration Ch(T) of the liver tissue of the region of interest ROI2. It is noted that the change of the xenon concentration Ch(T) in the region of interest ROI2 shown in FIG. 9 is changed approximately in synchronization with the change of the xenon concentration Ca(T) in the main artery 100 shown in FIG. 7. It is noted that the change of the xenon concentration Ch(T) in the region of interest ROI1 shown in FIG. 8 is changed with delay with respect to the change of the xenon concentration Ca(T) in the main artery 100 shown in FIG. 7.

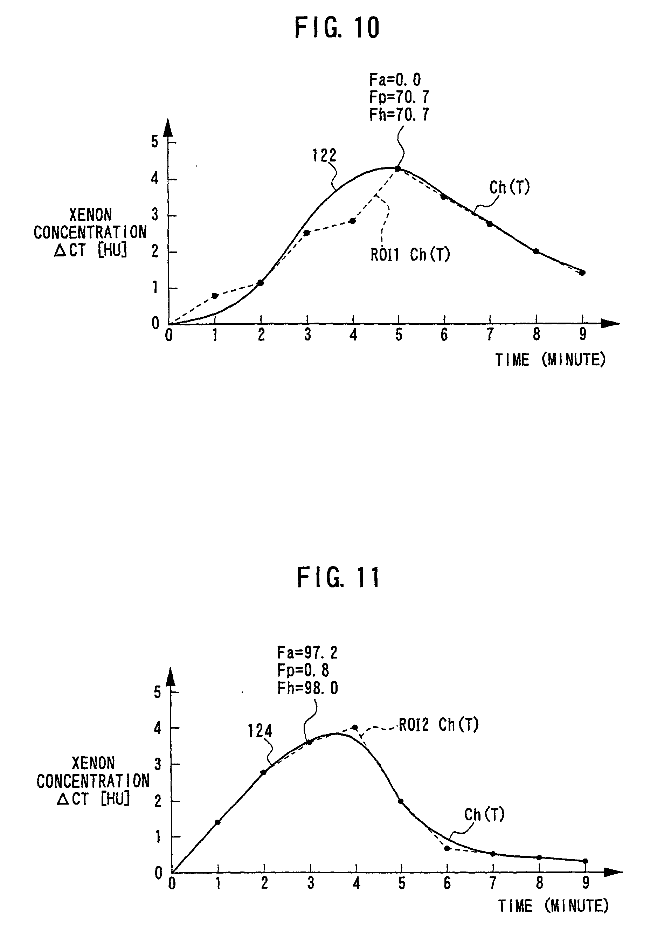

In FIG. 10, the reference numeral 122 indicates an approximate characteristic in which the xenon concentration Ch(T) in the region of interest ROI1 is determined with reference to the expression (2) by means of the least square method.

On the other hand, in FIG. 11, the reference numeral 124 indicates an approximate characteristic in which the xenon concentration Ch(T) in the region of interest ROI2 is determined with reference to the expression (2) by means of the least square method. FIGS. 7 to 11 are displayed on the display unit 56.

The approximate characteristic 122 shown in FIG. 10 is determined while assuming that the portal blood flow amount Fp is Fp = 70.7, the hepatic arterial blood flow amount Fa is Fa = 0.0, and the hepatic venous blood flow amount Fh is Fh = 70.7.

The approximate characteristic 124 shown in FIG. 11 is determined while assuming that the portal blood flow amount Fp is Fp = 0.8, the hepatic arterial blood flow amount Fa is Fa = 97.2, and the hepatic venous blood flow amount Fh is Fh = 98.

According to the approximate characteristic 122 shown in FIG. 10, it is understood that almost all of those flowing through the region of interest ROI1 are the hepatic arterial blood flow amount Fa. On the other hand, according to the approximate characteristic 124 shown in FIG. 11, it is understood that almost all of those flowing through the region of interest ROI2 are the portal blood flow amount Fp.

As described above, the hepatic arterial blood flow amount Fa and the portal blood flow amount Fp can be determined in a divided manner for each of the picture elements (each of the tissues) of the liver 54 of the specimen 12.

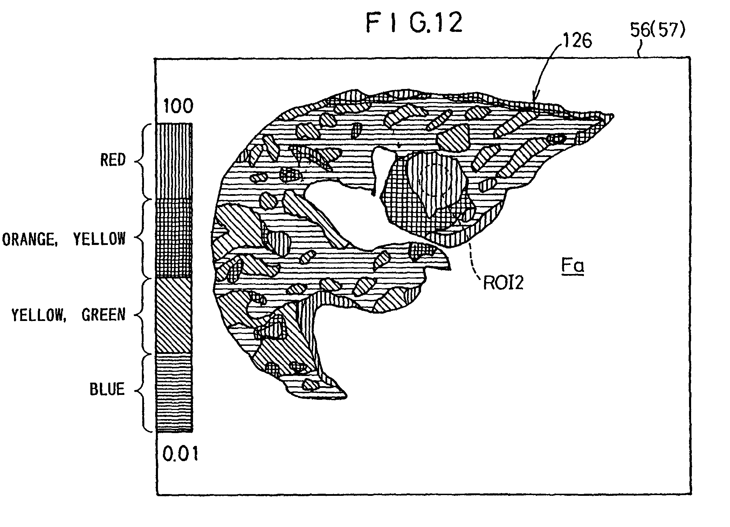

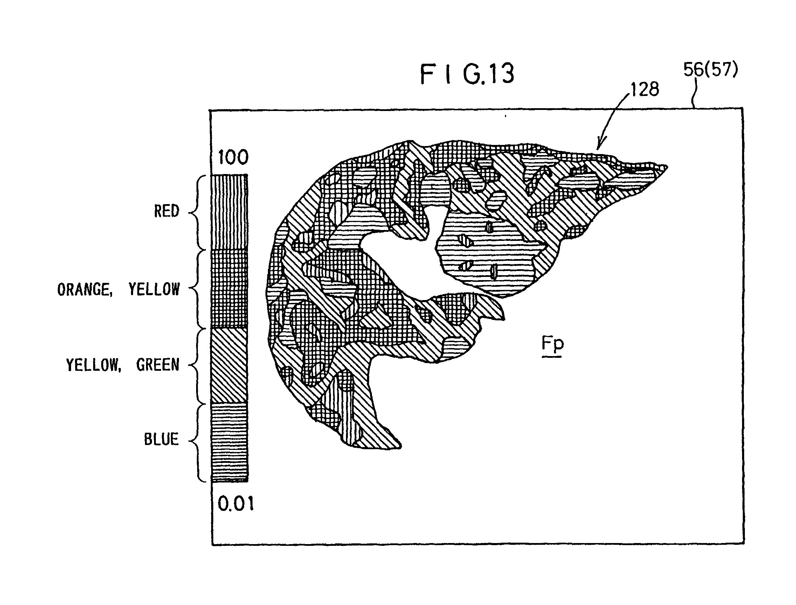

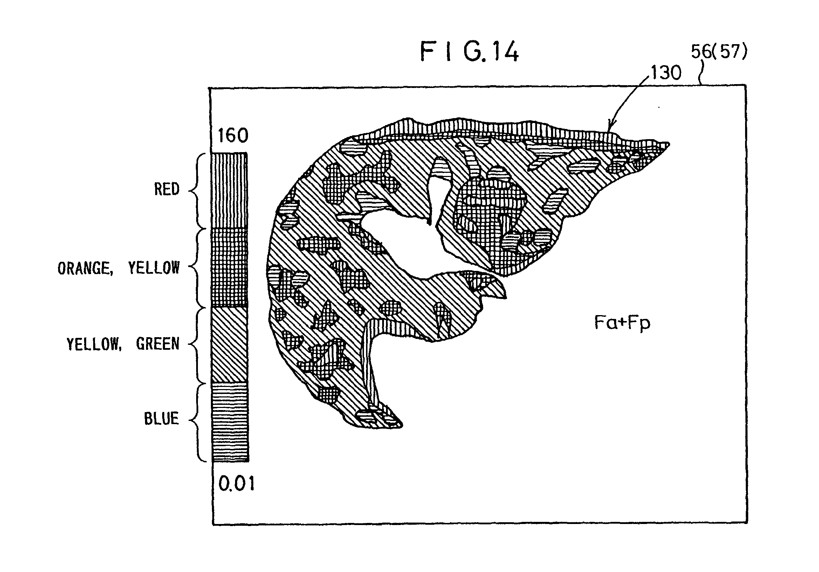

FIG. 12 schematically and illustratively shows a color-displayed map 126 of the hepatic arterial blood flow amount Fa (also referred to as "arterial map"). In FIG. 12, the indication ranging from a value of 0.01 to a value of 100 depicted at the left end indicates the value of the blood flow amount [cc/100 cc-tissue/min]. According to the map of the hepatic arterial blood flow amount Fa shown in FIG. 12, it is understood at a glance that almost all portions corresponding to the region of interest ROI2 are red, and almost all of those flowing through the tissue of the region of interest ROI2 are only the hepatic arterial blood flow amount Fa. It is postulated that any abnormal tissue such as malignant tumor exists in the site of the region of interest ROI2. FIG. 13 shows a color-displayed map 128 of the portal blood flow amount Fp (also referred to as "portal map"). FIG. 14 shows a color-displayed map 130 of the hepatic venous blood flow amount Fh (also referred to as "hepatic blood flow map").

The arterial map 126, the portal map 128, and the hepatic blood flow map 130 concerning the liver 54 have not been hitherto present. It is judged that extremely interesting results are obtained from a viewpoint of diagnosis.

According to the embodiment described above, the mixed gas supply apparatus 16 and the X-ray CT system 14 can be used to measure the hepatic arterial blood flow amount Fa and the portal blood flow amount Fp of the liver 54 of the specimen 12, and it is possible to make the color display in relation to the hepatic arterial blood flow amount Fa and the portal blood flow amount Fp corresponding to the results of the measurement.

In the embodiment described above, the completion of the xenon gas inhalation process is judged on the basis of the saturation of the xenon concentration in the expiration gas (see the step S8 in the flow chart shown in FIG. 3), and the completion of the xenon gas-washing process performed by making change into the inhalation of air is judged on the basis of the fact that the xenon concentration is not more than the predetermined value (see the step S11 in the flow chart shown in FIG. 3). However, the inventor of this application also have found out the fact that the time management is available such that the inhalation process is completed for about 4 minutes, and the washing process is completed for about 5 minutes, in order to measure the hepatic arterial blood flow amount Fa and the portal blood flow amount Fp of the liver 54 of the specimen 12.

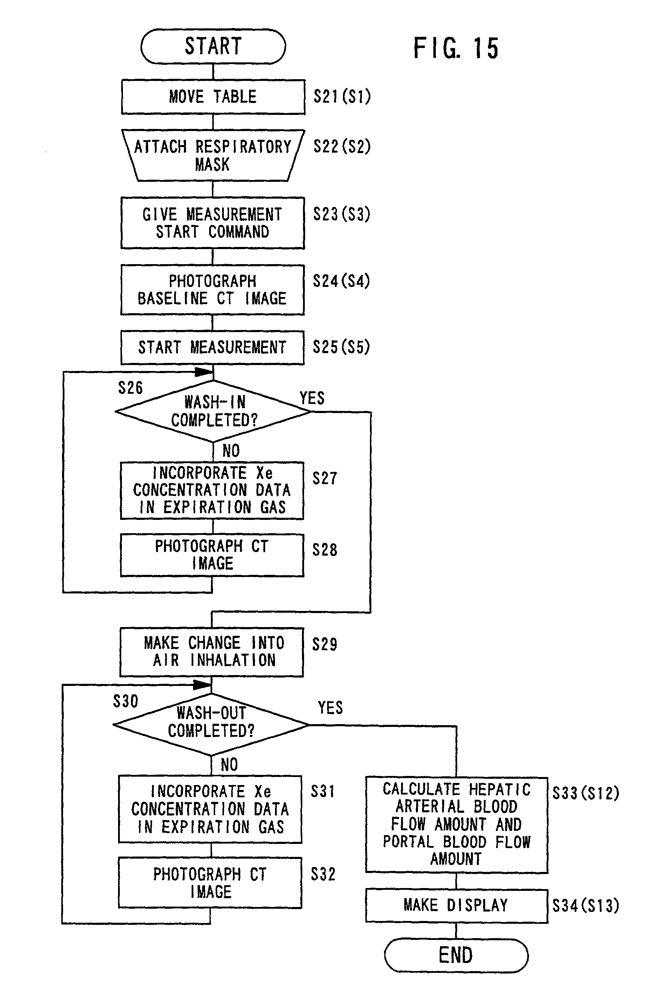

FIG. 15 shows a flow chart in which the measurement is managed along with time. Explanation will be made below with reference to the flow chart. In the flow chart shown in FIG. 15, the process steps corresponding to the process steps in the flow chart shown in FIG. 3 are indicated by parentheses affixed to the flow chart shown in FIG. 15.

At first, as shown in FIG. 4, the movable table 22 is moved in the direction of the arrow B in a state in which the specimen 12 is placed on the specimen-placing stand 24. The movable table 22 is stopped at a position at which the liver 54 of the specimen 12 can be photographed to obtain the tomographic image (step S21).

Subsequently, as shown in FIG. 4, the respiratory mask 44 is attached so that the mouth and the nose of the specimen 12 are covered therewith (step S22).

Subsequently, a measurement start command is generated (step S23), and the so-called baseline CT image is photographed by using the X-ray CT system 14 (step S24). The mixed gas of the xenon gas and the oxygen gas is fed from the mixed gas supply apparatus 16 to the specimen 12. Thus, the inhalation process (Wash-in) is started to begin the measurement (step S25). At the point of time of the start of the measurement, an unillustrated timer (time-measuring unit) contained in the control unit 20 starts the time measurement for a preset time of 4 minutes.

The completion of the inhalation process is confirmed by confirming whether or not the time measurement for 4 minutes is completed (step S26). The xenon concentration in the expiration gas is measured at every 40 msec until the completion (step S27). The CT image is photographed four times in total at about every 1 minute, i.e., at 1 minute, 2 minutes, 3 minutes, and 4 minutes (step S28).

When the time measurement for 4 minutes is completed (step S26: YES), the air is started to be supplied to the specimen 12 in place of the mixed gas (step S29).

Accordingly, the timer is operated to start the time measurement for a preset time of 5 minutes so that the washing process (Wash-out) for the xenon gas is started.

Until the completion of the time measurement for 5 minutes (step S30), the xenon concentration in the expiration gas is measured at every 40 ms (step S31). The CT image is further photographed five times in total at about every 1 minute, i.e., at 5 minutes, 6 minutes, 7 minutes, 8 minutes, and 9 minutes as counted from the start of the measurement in the step S25 (step S32).

The washing process is completed (step S30: YES) at the point of time at which the time measurement for 5 minutes is completed, i.e., at the point of time at which the time measurement for 9 minutes is completed in total from the start of the measurement in the step S25.

Subsequently, the hepatic arterial blood flow amount Fa and the portal blood flow amount Fp are calculated by using the expression (5) described above in accordance with the least square method (step S33) for each picture element of the CT image, from the xenon concentration Ch(T) in the liver tissue deduced from the CT image data photographed in the steps S28 and S32 and the xenon gas concentration Ca(T) in the arterial blood flow deduced from the xenon gas concentration data in the expiration gas measured in the steps S27 and S31.

Thus, for example, the color-displayed map (arterial map) 126 of the hepatic arterial blood flow amount Fa shown in FIG. 12, the color-displayed map (portal map) 128 of the portal blood flow amount Fp shown in FIG. 13, and the color-displayed map (hepatic blood flow map) 130 of the hepatic venous blood flow amount Fh shown in FIG. 14 are displayed in the same manner as in the step S13 to be used for diagnosis (step S34).

As explained above, it is noticed that when the xenon gas as an inert gas is introduced into the artery through the lung of the specimen, the xenon concentration in the liver tissue is determined from the xenon concentration in the hepatic arterial blood flow and the xenon concentration in the portal blood flow. The change in the xenon concentration in the liver tissue in the specimen is measured (detected) by making decomposition into the picture elements by using the X-ray CT system so that the hepatic arterial blood flow amount and the portal blood flow amount are determined for each of the picture elements obtained by the decomposition. Accordingly, it is possible to measure (detect) the portal blood flow amount and the arterial blood flow amount of each of the tissues of the liver. Further, the liver tissue concerning each of the picture elements can be easily distinguished whether it is a tissue in which the portal blood flow dominantly flows or it is a tissue in which the hepatic arterial blood flow dominantly flows.

The tomographic image of the liver, which is displayed by using the picture elements obtained by the X-ray CT system, is displayed (preferably displayed as different color illustrations) while substantially making separation into the map of the hepatic arterial blood flow and the map of the flow of the portal blood flow on the basis of the result of the distinction obtained as described above. Thus, the possibility is obtained to contribute to the diagnosis of the liver based on the hepatic blood flow.

The hepatic arterial blood flow amount and the portal blood flow amount can be measured in the form which is suitable for the diagnosis. Further, according to the present invention, it is possible to easily detect the portal blood flow amount and the hepatic arterial blood flow amount for each of the tissues of the liver. Furthermore, the tissue of the liver can be displayed (preferably displayed as color illustrations) while making separation into the tissue through which the portal blood flow flows and the tissue through which the arterial blood flow flows.

**Claims**

1. An apparatus for measuring a hepatic blood flow amount, comprising:

a gas inhalator (16) for supplying a mixed gas of xenon gas and oxygen gas to a lung of a specimen (12);
an X-ray CT system (14) for detecting a change in xenon concentration in a tissue of a liver of said specimen while making decomposition into picture elements;
a control unit (20) for controlling said gas inhalator and said X-ray CT system, wherein said control unit is used to operate said gas inhalator so that said mixed gas is inhaled by said specimen for a certain period of time, and determine a hepatic arterial blood flow amount (Fa) and a portal blood flow amount (Fp) concerning said respective picture elements obtained by said decomposition from picture element data detected by said decomposition while referring to an expression in which said xenon concentration in said liver tissue is determined by a xenon concentration in a hepatic arterial blood flow and a xenon concentration in a portal blood flow; and
a display unit (56),

wherein said control unit is operated such that a tomographic image of said liver represented by said picture elements obtained by said X-ray CT system is displayed on said display unit while dividing a display form into a map (126) to represent said hepatic arteria) blood flow and a map (128) to represent said portal blood flow, on the basis of said hepatic arterial blood flow amount and said portal blood flow amount determined as defined above.

2. The apparatus according to claim 1, wherein assuming that said xenon concentration in said liver tissue is Ch(T) provided that T represents a measurement time, said hepatic arterial blood flow amount is Fa, said xenon concentration in said hepatic arterial blood flow is Ca(t) and Ca(x) provided that t and x represent passed times, a hepatic venous blood flow amount is Fh provided that there is given Fh = Fa + Fp, a ratio between a xenon concentration in tissues of portal internal organs and a xenon concentration in blood is $\lambda$p, and a ratio between said xenon concentration in said tissue of said liver and said xenon concentration in blood is $\lambda$h, said expression provides said xenon concentration Ch(T) in said liver tissue as calculated as follows :

$$Ch(T) = Fa \int Ca(t) \bullet exp\{(-Fh/\lambda h)(T-t)\}dt$$

$$+ Fp(Fp/\lambda p) \int [\int Ca(x) \bullet exp\{(-Fp/\lambda p)(t-x)\}dx] \bullet$$

$$exp\{(-Fh/\lambda p)(T-t)\}dt$$

wherein an interval of a definite integral of a first term on a right side is [0, T], an interval of a definite integral for an infinitesimal time dx is [0, t], and an interval of a definite integral for an infinitesimal time dt is [0, T] in a double integral of a second term on said right side.

3. The apparatus according to claim 1 or 2, wherein said mixed gas is a mixed gas of a ratio of 30 % of xenon gas and 70 % of oxygen.

4. The apparatus according to claim 1 or 2, wherein an inhalation period, which is a time for supplying said mixed gas to said lung of said specimen for said certain period of time, is about 4 minutes.

5. The apparatus according to claim 4, wherein a washing period after said inhalation period of about 4 minutes is about 5 minutes.

6. The apparatus according to any one of claims 1 to 5 wherein said control unit judges that a portion, in which a value of a ratio [hepatic arterial blood flow amount/portal blood flow amount] between said hepatic arterial blood flow amount (Fa) and said portal blood flow amount (Fp) concerning said respective determined picture elements is about 1/3, is a normal tissue, and it judges that a portion, in which said value of said ratio is deviated, is an abnormal tissue.

7. The apparatus according to claims 1 to 6 wherein said display display form on said display unit is a display form to make display in which a color of said map to represent said hepatic arterial blood flow is different from that of said map to represent said portal blood flow.

**Patentansprüche**

1. Vorrichtung zum Messen einer hepatischen Blutstrommenge, umfassend:

einen Gasinhalator (16) zum Zuführen eines Gasgemisches aus Xenongas und Sauerstoffgas zu einer Lunge eines Probanden (12);

eine Röntgen-CT-System (14) zum Erfassen einer Änderung der Xenonkonzentration in einem Gewebe einer Leber des Probanden, während eine Zerlegung in Bildelemente vorgenommen wird;

eine Steuereinheit (20) zum Steuern des Gasinhalators und des Röntgen-CT-Systems, wobei die Steuereinheit dazu eingesetzt wird, den Gasinhalator derart zu betreiben, daß das Gasgemisch von dem Probanden für eine gewisse Zeitspanne inhaliert wird, und zum Bestimmen einer hepatischen Arterien-Blutstrommenge (Fa) und einer Pfortader-Blutstrommenge (Fp) bezüglich der jeweiligen Bildelemente, die durch die Zerlegung aus den Bildelementdaten erhalten wurden, die durch die Zerlegung erhalten wurden, während Bezug genommen wird auf einen Ausdruck, bei dem die Xenonkonzentration in dem Lebergewebe bestimmt wird durch eine Xenonkonzentration in einem hepatischen Arterien-Blutstrom und eine Xenonkonzentration in einem Pfortader-Blutstrom; und

eine Anzeigeeinheit (56),

wobei die Steuereinheit derart betrieben wird, daß ein tomographisches Bild der Leber, dargestellt durch die von dem Röntgen-CT-System erhaltenen Bildelemente, auf der Anzeigeeinheit angezeigt wird, während eine Anzeigeform unterteilt wird in eine Karte (126) zur Darstellung des hepatischen Arterien-Blutstroms und eine Karte (128) zur Darstellung des Pfortader-Blutstroms, auf der Grundlage der hepatischen Arterien-Blutstrommenge und der Pfortader-Blutstrommenge, die in der oben genannten Weise ermittelt wurden.

2. Vorrichtung nach Anspruch 1, bei der unter der Annahme, daß die Xenonkonzentration im Lebergewebe Ch(T) beträgt, wobei T eine Meßzeit bedeutet, die hepatische Arterien-Blutstrommenge Fa ist, die Xenonkonzentration in dem hepatischen Arterien-Blutstrom Ca(t) und Ca(x) mit t und x als verstrichene Zeiten ist, eine hepatische venöse Blutstrommenge Fh mit der Beziehung Fh = Fa + Fp ist, ein Verhältnis zwischen einer Xenonkonzentration in Geweben von internen Pfortaderorganen und einer Xenonkonzentration in Blut λp beträgt und ein Verhältnis zwischen der Xenonkonzentration in dem Gewebe der Leber und der Xenonkonzentration in Blut λh beträgt, der Ausdruck die Xenonkonzentration Ch(T) im Lebergewebe gemäß folgender Berechnung liefert:

$$Ch(T) = Fa \int Ca(t) \cdot exp\{(-Fh/\lambda h)(T-t)\} \, dt$$

$$+ Fp(Fp/\lambda p) \int [\int Ca(x) \cdot exp\{(-Fp/\lambda p)(t-x)\} dx] \cdot$$

$$exp\{(-Fh/\lambda p)(T-t)\} dt$$

wobei ein Intervall eines definierten Integrals des ersten Terms auf der rechten Seite [0, T] beträgt, ein Intervall eines definierten Integrals für eine infinitesimale Zeit dx [0, t] beträgt, und ein Intervall für ein definiertes Integral einer infinitesimalen Zeit dt [0, T] in einem Doppelintegral eines zweiten Terms auf der rechten Seite beträgt.

3. Vorrichtung nach Anspruch 1 oder 2, bei der das Gasgemisch ein Gasgemisch in einem Verhältnis von 30% Xenongas und 70% Sauerstoff ist.

4. Vorrichtung nach Anspruch 1 oder 2, bei der eine Inhalationszeitspanne, bei der es sich um eine Zeit zum Zuführen des Gasgemisches zu der Lunge des Probanden während einer gewissen Zeitspanne handelt, etwa 4 Minuten beträgt.

5. Vorrichtung nach Anspruch 4, bei der eine Spülperiode nach der Inhalationszeitspanne von etwa 4 Minuten etwa 5 Minuten beträgt.

6. Vorrichtung nach einem der Ansprüche 1 bis 5, bei der die Steuereinheit beurteilt, daß ein Bereich, in welchem ein Wert eines Verhältnisses [hepatischer Arterien-Blutstrommenge/Pfortader-Blutstrommenge] zwischen der hepatischen Arterien-Blutstrommenge (Fa) und der Pfortader-Blutstrommenge (Fp) bezüglich der jeweiligen ermittelten Bildelemente etwa 1/3 beträgt, es sich um Normalgewebe handelt, und beurteilt, daß ein Bereich, in welchem der Wert des Verhältnisses abweicht, abnormales Gewebe ist.

7. Vorrichtung nach einem der Ansprüche 1 bis 6, bei der die Anzeigeform auf der Anzeigeeinheit eine Anzeigeform ist, bei der die Anzeige, in welcher eine Farbe der Karte den hepatischen Arterien-Blutstrom darstellt, sich unterscheidet von der Karte, mit der der Pfortader-Blutstrom dargestellt wird.

**Revendications**

1. Appareil de mesure du débit sanguin hépatique, comprenant :

un inhalateur de gaz (16) approvisionnant les poumons d'un sujet (12) en un mélange de gaz de xénon et d'oxygène ;

un tomodensitomètre (14) détectant les changements de concentration en xénon dans le tissu hépatique dudit sujet tout en produisant une décomposition de l'image par pixel ;
une unité de commande (20) contrôlant ledit inhalateur de gaz et ledit tomodensitomètre, dans lequel ladite unité de commande est utilisée pour faire fonctionner ledit inhalateur de gaz de façon à ce que ledit mélange de gaz soit inhalé par ledit sujet pendant un certain laps de temps, et pour déterminer un débit sanguin artériel hépatique (Da) et un débit sanguin portal (Dp) concernant lesdits pixels respectifs obtenus par ladite décomposition provenant des données de pixels détectées par ladite décomposition tout en se référant à une expression dans laquelle ladite concentration en xénon dans ledit tissu hépatique est déterminée par une concentration en xénon dans un débit sanguin artériel hépatique et une concentration en xénon dans un débit sanguin portal ; et
une unité d'affichage (56),

dans lequel ladite unité de commande fonctionne de telle sorte qu'une image tomographique dudit foie représentée par lesdits pixels obtenus par ledit tomodensitomètre apparaît sur ladite unité d'affichage tout en produisant une illustration différenciant une carte (126) représentant ledit débit sanguin artériel hépatique d'une carte (128) représentant ledit débit sanguin portal, sur la base dudit débit sanguin artériel hépatique et dudit débit sanguin portal déterminés comme défini ci-dessus.

2. Appareil selon la revendication 1, dans lequel partant du principe que ladite concentration en xénon dans ledit tissu hépatique est Ch(T) à condition que T représente un temps de mesure, ledit débit sanguin artériel hépatique est Da, ladite concentration en xénon dans ledit débit sanguin artériel hépatique est Ca(t) et Ca(x) à condition que t et x représentent des instants passés, un débit sanguin veineux hépatique est Dh à condition que soit donné Dh = Da + Dp, un rapport entre une concentration en xénon dans les tissus des organes internes portaux et une concentration en xénon dans le sang est $\lambda$p, et un rapport entre ladite concentration en xénon dans ledit tissu dudit foie et ladite concentration en xénon dans le sang est $\lambda$h, ladite expression propose de calculer ladite concentration en xénon Ch(T) dans ledit tissu hépatique comme suit :

$$Ch(T) = Da \int Ca\,(t) \bullet \exp\{(-Dh/\lambda h)(T-t)\}dt$$

$$+ \, Dp(Dp/\lambda p) \int [\int Ca(x) \bullet \exp\{(-Dp/\lambda p)(t-x)\}dx\} \bullet$$

$$\exp\{(-Dh/\lambda p)(T-t)\}dt$$

où un intervalle d'une intégrale définie d'un premier terme du côté droit est [0,T], un intervalle d'une intégrale définie pour un temps infinitésimal dx est [0,t], et un intervalle d'une intégrale définie pour un temps infinitésimal dt est [0,T] dans une double intégrale d'un second terme dudit côté droit.

3. Appareil selon la revendication 1 ou 2, dans lequel ledit mélange de gaz est un mélange gazeux d'un rapport de 30 % de xénon et 70 % d'oxygène.

4. Appareil selon la revendication 1 ou 2, dans lequel une période d'inhalation, qui est un temps d'approvisionnement desdits poumons dudit sujet en ledit mélange de gaz pendant ledit laps de temps, est d'environ 4 minutes.

5. Appareil selon la revendication 4, dans lequel une période de lavage après ladite période d'inhalation d'environ 4 minutes est d'environ 5 minutes.

6. Appareil selon l'une quelconque des revendications 1 à 5, dans lequel ladite unité de commande juge qu'une portion, dans laquelle une valeur d'un rapport [débit sanguin artériel hépatique/débit sanguin portal] entre ledit débit sanguin artériel hépatique (Da) et ledit débit sanguin portal (Dp) concernant lesdits pixels respectifs déterminés est environ 1/3, est un tissu normal, et juge qu'une portion, dans laquelle ladite valeur dudit rapport est déviée, est un tissu anormal.

7. Appareil selon les revendications 1 à 6 dans lequel ladite illustration apparaissant sur ladite unité d'affichage est une illustration dans laquelle la couleur de ladite carte représentant ledit débit sanguin artériel hépatique est différente de celle de ladite carte représentant ledit débit sanguin portal.

FIG.1

# FIG. 2

14 X-RAY CT SYSTEM    10

18 MAIN X-RAY CT SYSTEM

20 CONTROL UNIT

32

28 X-RAY TUBE

56 DISPLAY UNIT

16 MIXED GAS SUPPLY APPARATUS

12 SPECIMEN

50 COMPUTER

55 EXTERNAL STORAGE UNIT

30 DETECTOR

52 OPERATION CONSOLE

EP 1 036 542 B1

FIG. 3

START

MOVE TABLE — S1

ATTACH RESPIRATORY MASK — S2

GIVE MEASUREMENT START COMMAND — S3

PHOTOGRAPH BASELINE CT IMAGE — S4

START MEASUREMENT — S5

INCORPORATE IMAGE DATA — S6

CALCULATE XENON CONCENTRATION IN TISSUE — S7

S8
SATURATED STATE ACHIEVED?
NO
YES

S9
WASHING PROCESS COMPLETED?
YES
NO

EXECUTE WASHING PROCESS — S10

S11
XENON CONCENTRATION NOT MORE THAN PREDETERMINED VALUE?
NO
YES

CALCULATE HEPATIC ARTERIAL BLOOD FLOW AMOUNT AND PORTAL BLOOD FLOW AMOUNT — S12

MAKE DISPLAY — S13

END

14

# FIG.4

EP 1 036 542 B1

# FIG. 5

FIG.6

FIG. 7

FIG. 8

FIG. 9

# FIG. 10

Fa=0. 0
Fp=70. 7
Fh=70. 7

122

XENON CONCENTRATION $\triangle CT$ [HU]

Ch(T)

ROI1 Ch(T)

TIME (MINUTE)

# FIG. 11

Fa=97. 2
Fp=0. 8
Fh=98. 0

124

ROI2 Ch(T)

XENON CONCENTRATION $\triangle CT$ [HU]

Ch(T)

TIME (MINUTE)

# F I G.12

56(57)

126

100

RED

ORANGE, YELLOW

YELLOW, GREEN

BLUE

0.01

ROI2

Fa

EP 1 036 542 B1

F I G.13

56(57)

128

100

RED

ORANGE, YELLOW

YELLOW, GREEN

BLUE

0.01

Fp

EP 1 036 542 B1

# F I G. 14

56(57)

130

160

RED

ORANGE, YELLOW

YELLOW, GREEN

BLUE

0.01

Fa+Fp

EP 1 036 542 B1

EP 1 036 542 B1

FIG. 15

START

MOVE TABLE — S21(S1)

ATTACH RESPIRATORY MASK — S22(S2)

GIVE MEASUREMENT START COMMAND — S23(S3)

PHOTOGRAPH BASELINE CT IMAGE — S24(S4)

START MEASUREMENT — S25(S5)

S26 WASH-IN COMPLETED? — YES

NO

INCORPORATE Xe CONCENTRATION DATA IN EXPIRATION GAS — S27

PHOTOGRAPH CT IMAGE — S28

MAKE CHANGE INTO AIR INHALATION — S29

S30 WASH-OUT COMPLETED? — YES

NO

INCORPORATE Xe CONCENTRATION DATA IN EXPIRATION GAS — S31

PHOTOGRAPH CT IMAGE — S32

CALCULATE HEPATIC ARTERIAL BLOOD FLOW AMOUNT AND PORTAL BLOOD FLOW AMOUNT — S33(S12)

MAKE DISPLAY — S34(S13)

END

23